(19) Europäisches Patentamt
European Patent Office
Office européen des brevets

(11) **EP 2 844 310 B1**

(12) **EUROPEAN PATENT SPECIFICATION**

(45) Date of publication and mention of the grant of the patent:
**06.09.2017 Bulletin 2017/36**

(21) Application number: **13729450.0**

(22) Date of filing: **02.05.2013**

(51) Int Cl.:
*A61L 27/60* (2006.01)    *C08B 37/00* (2006.01)
*A61L 27/38* (2006.01)

(86) International application number:
**PCT/IB2013/053475**

(87) International publication number:
**WO 2013/164782 (07.11.2013 Gazette 2013/45)**

(54) **SHAPE-MEMORY CROSS-LINKED POLYSACCHARIDES**

VERNETZTE FORMGEDÄCHTNISPOLYSACCHARIDE

POLYSACCHARIDES RÉTICULÉS À MÉMOIRE DE FORME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2012 IT MI20120732**

(43) Date of publication of application:
**11.03.2015 Bulletin 2015/11**

(73) Proprietor: **Medical and Biotechnological Services Srl**
**in abbreviated form M.B.S. Srl**
**83100 Avellino (IT)**

(72) Inventors:
• **DE ROSA, Mario**
**I-83100 Avellino (IT)**
• **SCHIRALDI, Chiara**
**I-83100 Avellino (IT)**
• **LA GATTA, Annalisa**
**I-83100 Avellino (IT)**

(74) Representative: **Minoja, Fabrizio**
**Bianchetti Bracco Minoja S.r.l.**
**Via Plinio, 63**
**20129 Milano (IT)**

(56) References cited:
**WO-A2-2011/023355    CN-A- 102 321 258**
**US-A1- 2010 028 435**

## Description

The present invention relates to a process for the preparation of crosslinked shape-memory polysaccharides, which comprises crosslinking the polysaccharide in a non-solvent system for the polysaccharide.

## State of the art

Hyaluronic acid (HA) is the glycosaminoglycan polysaccharide which is currently attracting the greatest application interest in the biomedical field. HA is a negatively-charged linear-chain polysaccharide, consisting of the repetition of n disaccharide units (-4GlcUAβ1-3GlcNAcβ1-), wherein D-glucuronic acid (GlcUA) and N-acetyl-D-glucosamine (Glc-NAc) are bonded with alternating glycoside bonds β-1,3 and β-1,4.

HA is a polysaccharide that is highly soluble in water, and HA solutions exhibit non-Newtonian viscoelastic behaviour.

HA is one of the basic ingredients of extracellular matrix (ECM). In vertebrates, HA has a wide variety of functions: in the skin it guarantees tissue hydration, and in the cartilage HA binds to proteoglycans to regulate the water and ion content, to stabilise the physical (viscoelastic) properties of the tissue and the cell-substrate interactions. The average molecular weight of the HA in the synovial fluid and umbilical cord is 3,000-4,000 KDa. The biological response elicited by HA by interaction with the receptors present on the cell membranes depends mainly on the molecular weight of the polysaccharide chains; in particular the accumulation of low-molecular-weight HA represents an early sign of alteration of the ECM, which activates tissue repair responses, while a preponderance of high-molecular-weight HA encodes for a situation of good homeostasis.

Due to its natural presence in the tissues, HA does not trigger immune responses when implanted in the body; for this reason, and due to the unique rheological and lubricant properties described below, HA is one of the most versatile, interesting biomaterials currently available for applications in the biomedical field.

**Hydrophilicity and rheological properties -** In aqueous solution the HA molecules trap large amounts of water (about 1000 times the weight of HA), taking on the form of extended spirals stabilised by the hydrogen bonds between the hydroxyl groups along the chain. Said chains envelop one another even at very low concentrations; even very dilute HA solutions therefore have high viscosity levels, but dependent on the shear rate.

**Lubricant properties -** The extraordinary rheological properties of HA solutions make this compound an ideal candidate as lubricant in the biological field. This is one of the actions performed by HA in the body, wherein it separates the surfaces of the different tissues which, due to its presence, slide against one another. The joints of the human body are an example of this type of behaviour.

**Signalling action and biorevitalising properties -** When HA is implanted in the body, it elicits differentiated biological responses in the cells and tissues, which depend on the molecular weight of the material and give rise to revitalisation of the biological structures.

The natural presence of HA in the body and its ability to retain water have led to its use in a variety of therapeutic applications, such as treatment of arthritic disorders, use as a substitute for the vitreous humour, prevention of adhesions after surgery, protection of wounds during healing, viscosupplementation treatments in the joints, and use in aesthetic medicine to correct blemishes associated with skin aging.

One of the major limitations in all these therapeutic applications of HA is its rapid degradation due to the action of different enzymes, such as hyaluronidase, glucuronidase and glucosidase, or to radical processes that alter its rheological properties and reduce its residence *times in vivo*.

An effective approach to delaying the degradation of HA *in vivo* is to modify its structure with a crosslinking agent which, by anchoring different polysaccharide chains to one another or creating loops on the same chain, limits the degradation processes responsible for the short half-life of this polysaccharide *in vivo.*

In particular, crosslinking HA involves the formation of inter- or intra-chain covalent bonds which are formed by reacting the polysaccharide with multifunctional molecules able to form covalent bonds with the hydroxyl and carboxyl moieties present on HA. According to the chemical nature of the crosslinking agent, the stoichiometry and the reaction conditions, different HA crosslinking processes can be generated, to obtain a wide variety of products with differentiated chemical, biological and rheological properties.

One of the most common crosslinking techniques, in particular in the case of HA, is the reaction with diepoxides in a alkaline medium with the formation of ether bonds. The most widely used diepoxide is 1,4-butanediol diglycidyl ether (BDDE). This type of crosslinking offers the advantage of great stability of the ether bonds formed and absence of toxicity of the crosslinked product, which retains all the biocompatibility characteristics typical of HA.

The use of diepoxides in the crosslinking of polysaccharides, in particular HA, is extensively reported in the patent literature. In most cases (US 4,963,666, US 5,827,937, KR 10-2005-0059521, US 6,921,819, US 2005/0281880, US 20060105022, US 2007/0026070, US 2007/0196426, US 6,630,167) the crosslinking reaction involves solubilisation of HA in NaOH followed by its crosslinking in homogeneous phase using BDDE.

As can be seen from table 1, which compares the various methodologies at the basis for the above-mentioned patent documents, all the crosslinking methods based on the use of BDDE in an alkaline medium which have been developed to date fail to solve the problem of a crosslinking process that maintains (retains) the original shape of the material. In all the cases reported in the scientific and patent literature, the crosslinking reaction with BDDE in a basic medium uses solvent systems for HA that modify the original shape of the material to be crosslinked.

Maintenance of the 3D structure of the material during the crosslinking process is of crucial importance when, for example, particulates, membranes, spongy structures or tissue engineering scaffolds on HA are to be crosslinked.

The importance of developing a crosslinking strategy that maintains the three-dimensional structure of HA during the crosslinking process, which is therefore known as "shape memory", is evident in view of the factors set out above.

Table 1 - Description of HA crosslinking methods with diepoxides (BDDE) reported in US4,963,666, US 5,827,937, KR 10-2005-0059521, US 6,921,819, US 2005/0281880, US 20060105022, US 2007/0026070, US 2007/0196426 and US 6,630,167. All the data are standardised for a process involving crosslinking 10g of HA.

| Reaction on 10 g of HA with BDDE as crosslinking agent | BDDE/ HA (% p/p) | $H_2O$ (mL) | h | (°C) | REFERENCE |
|---|---|---|---|---|---|
| HA is dissolved in 0.5% NaOH and crosslinked with BDDE (epoxy-activation); the excess BDDE is removed by dialysis for 24 h, and crosslinked at the drying step for 48h at 25°C. A film that dissolves in 48 h is obtained. | 1 | 300 | 12 | 25 | US 4,963,666 |
| Reaction method that initially leads to an activated HA, blocking the crosslinking with a dilution process before the HA gels, and subsequently completes the crosslinking process by concentrating the reaction mixture. HA is dissolved in 1% NaOH, and BDDE is added. After the first reaction at 40°C the mixture is diluted 10 times, neutralised and concentrated in a rotavapor. A continuous gel is obtained. | 0,20 | 100 | 4 | 40 | US 5,827,937 |
| The method requires the addition to the basic solution containing HA and BDDE of a large volume of ethanol or acetone that precipitates HA, which continues to crosslink even after precipitation. The HA (5-15% w/w) is dissolved in 4-19% NaOH to which BDDE is added; the mixture is then poured into ethanol or acetone to obtain a precipitate. The mixture is neutralised and washed with $H_2O$, and a granulated or fibrous material is obtained. | 2,4 | 400 | 24 | 25-50 | KR 10-2005-0059521 |
| The method claims that the crosslinking process takes place during hydration of HA. HA is hydrated with 68 g of 1% NaOH containing BDDE. A continuous gel is obtained which is neutralised with phosphate buffer and homogenised. | 6,8 | 67 | 3 | 50 | US 6,921,819 |
| The method requires the reaction to be performed in a reactor in which all steps of the 3-day process are performed. HA is dissolved in 1% NaOH and BDDE is added. A continuous gel is obtained. | 60-100 | 100 | 4 | 50 | US 2005/0281880 |
| Reaction method at a high HA concentration (>20%), with rotation-revolution mixing. The HA is hydrated with 22.5mL of 0.2N NaOH containing BDDE, rotation-revolution mixed for 5 min and left at ambient temperature for 24 h. A continuous gel is obtained. | 0,5 | 22,5 | 24 | 25 | US 20060105022 |

(continued)

| Reaction on 10 g of HA with BDDE as crosslinking agent | BDDE/ HA (% p/p) | H$_2$O (mL) | h | (°C) | REFERENCE |
|---|---|---|---|---|---|
| The method involves a first crosslinking step in the basic solution and a subsequent step concomitant with drying under vacuum of the reaction mixture. The HA is dissolved in 1% NaOH to which BDDE is added. After 2 h at 45°C the mixture is dried under vacuum at 40°C for 1.5 h. It is then washed three times with 500 mL of isopropyl alcohol (IPA)-H$_2$O 6:4 for 22 h; the solvent is removed and the mixture is washed with 500 mL of 1.3% CH$_3$COOH in H$_2$O for 35 min, 1L IPA, 500 mL IPA-H$_2$O 6:4; 2L IPA, 2L IPA-H$_2$O 8:2, and 1L IPA. | 2,5 | 250 | 2 | 45 | US 2007/0026070 |
| The method involves a double crosslinking reaction: HA is dissolved in 1% NaOH in the first, and BDDE is added in the second. Another 200 mL of 0.5% HA at pH 11 is added. A continuous gel is obtained. | 0,85 | 100 | 9 6 | 25 25 | US 2007/0196426 |
| A method for preparing crosslinked HA sponges obtained by freeze-drying aqueous solutions of HA and crosslinking the lyophilisate is disclosed. An aqueous solution of HA is then introduced into the crosslinked sponge to produce an anti-adhesion barrier for surgical use. The patent generically claims all types of crosslinking, but does not give any examples with BDDE or other di- epoxides. | | | | | US 6,630,167 |

## Description of the invention

It has now been found that the crosslinking reaction of HA and other polysaccharides such as chondroitin, chondroitin sulphate, chitosan and polydextran can be performed with polyfunctional epoxides, operating directly in a non-solvent system for the polysaccharide so as to obtain a crosslinked polysaccharide that retains the 3D characteristics of the starting polysaccharide ("shape memory").

The process is more advantageous than the prior art, involving low reaction volumes, shorter times for synthesis and purification of the crosslinked material, less onerous synthesis costs, and the unique possibility of maintaining the 3D shape of the material ("shape memory") during crosslinking.

As discussed above, the crosslinking reaction of polysaccharides, in particular HA, in a basic medium, with polyfunctional epoxy reagents, currently represents the first-choice strategy in the production of crosslinked HA, due to the high chemical and biological stability of the ether bond and the total biocompatibility of this type of material after implantation.

The crucial problem of the crosslinking reaction of HA with polyfunctional epoxides is the need to operate in a strongly basic medium, pH > 12, for times amounting to several hours at temperatures ranging between 20 and 70°C. These conditions cause, in parallel with the intra- and intermolecular crosslinking process, hydrolysis of the glycoside bonds present on the polysaccharide chain, leading to a reduction in its molecular weight. The competition between these two processes determines the structural characteristics of the crosslinked polysaccharide matrix formed, which is substantially characterised by the presence in the crosslinked system of linear polymer segments which are significantly shorter than those originally used. The identification of reaction conditions that minimise the hydrolysis process compared with the crosslinking process is therefore particularly important.

In the methods known to date, HA has always been crosslinked in solvent systems for HA, usually aqueous solutions of NaOH, giving rise to actual solutions or hydrated pastes which, when the reaction is complete, consist of a continuous gels which can be reduced by mechanical treatment to small particles, generally between 50 and 500 $\mu$m. In particular in the case of the reaction in solution (US 4,963,666, US 5,827,937, US 6,921,819, US 2005/0281880, US 2007/0026070, US 2007/0196426 and US 6,630,167), large reaction volumes are required due to the high viscosity of the hyaluronic acid solutions, which make the synthesis process complex and very expensive.

However, the use of hydrated hyaluronic acid pastes (US 20060105022) creates problems associated with the strong consistency of said masses, which are difficult to mix homogeneously, especially on an industrial scale.

The process according to the invention comprises crosslinking the polysaccharide in a non-solvent system for the polysaccharide in the presence of quaternary ammonium bases and a polyfunctional epoxide.

The process can be used to crosslink hyaluronic acid, chondroitin and chondroitin sulphate, preferably hyaluronic acid.

The non-solvent system consists of mixtures of organic solvents and water with a volume ratio between organic solvent and water $\geq 2$.

Examples of suitable organic solvents are ketones, alcohols and ethers miscible with water. Acetone is particularly preferred.

The reaction must take place at pH values > 12, which can be reached with quaternary ammonium bases but not with inorganic hydroxides such as alkali metal and alkaline earth metal hydroxides.

Examples of quaternary ammonium bases are tetrabutyl ammonium hydroxide, tetrapropyl ammonium hydroxide, tetraethyl ammonium hydroxide, tetramethyl ammonium hydroxide, benzyl tributyl ammonium hydroxide, benzyl tripropyl ammonium hydroxide, benzyl triethyl ammonium hydroxide, benzyl trimethyl ammonium hydroxide, methyl tributyl ammonium hydroxide, methyl tripropyl ammonium hydroxide, methyl triethyl ammonium hydroxide, phenyl tributyl ammonium hydroxide, phenyl tripropyl ammonium hydroxide, phenyl triethyl ammonium hydroxide, phenyl trimethyl ammonium hydroxide, dodecyl trimethyl ammonium hydroxide, tetradecyl trimethyl ammonium hydroxide, hexadecyl trimethyl ammonium hydroxide, octadecyl trimethyl ammonium hydroxide and choline hydroxide. Said bases are preferably used at concentrations ranging between 0.125 and 1.3 M. Benzyl trimethyl ammonium hydroxide is particularly preferred.

The polyfunctional epoxy crosslinking agent is selected from 1,3-butadiene diepoxide, 1,2,7,8-diepoxyoctane, epoxy 1,5-hexadiene, ethylene glycol diglycidyl ether, 1,4-butanediol diglycidyl ether, 1,6-hexanediol diglycidyl ether, polyethylene glycol diglycidyl ether, polypropylene glycol diglycidyl ether and bisphenol A diglycidyl ether. 1,4-butanediol diglycidyl ether, polyethylene glycol and polypropylene glycol diglycidyl ether are preferred.

The molar ratio of crosslinker respect to reactive polysaccharide groups affects the reaction yields and the characteristics of the crosslinked HA. The higher said ratio, the greater the degree of crosslinking, the percentage yield of insoluble crosslinked HA and its stability to attack by hyaluronidase and free radicals.

The molar ratio of epoxy groups present in the crosslinking agent to the functional polysaccharide groups able to react with the epoxy groups is $\leq 1$.

The reaction can be conducted at temperatures between 30 and 80°C, for reaction times between 1.5 and 100 h, preferably between 2 and 16 h. The yields of crosslinked HA linearly increase with the increase in reaction temperature up to T = 60°C. At higher temperatures the reaction yield declines due to the prevalence of hydrolysis processes associated with the basic reaction medium. Increasing reaction times cause an increase in the yields of crosslinked insoluble hyaluronic acid. Said increase is particularly marked in the first 3 h, when about 70% of the crosslinked HA is formed, taking the amount formed after 16 h as 100%. Reaction times of 3-16 h are therefore preferred, preferably between 3 and 8 hours, because they represent the best compromise between the reaction yield and the process times.

The downstream purification process of crosslinked HA initially involves arresting the crosslinking reaction by adjusting the pH of the reaction mixture to 7.4 by adding concentrated $H_3PO_4$. The next step involves purification of the crosslinked HA by removing the liquid phase containing the reagents and by-products of the reaction, and successive washings with aqueous-organic solutions. Ethanol, acetone, tetrahydrofuran and dimethylformamide, preferably ethanol and acetone, can be used as organic solvents mixed with water. The percentage of water present in the washing solution affects the degree of swelling of the crosslinked polysaccharide; a solution of about 1:1 (v/v) is preferably used. The saturation of the washing solution with inorganic salts, mainly alkali metal or alkaline earth metal chlorides such as NaCl, KCl, $CaCl_2$, etc., in the first steps of the purification process, allows the quaternary ammonium ion deriving from the organic base used in the reaction to be moved, in order to obtain the desired salt (crosslinked sodium, potassium or calcium hyaluronate, etc.). The removal of the quaternary ammonium ion is spectrophotometrically monitored. The last washing steps are performed with water/organic solvent solutions not saturated with the salt. When the conductivity reaches values of a few tens of $\mu$S/cm, the crosslinked solid phase is recovered, and can be dried by freeze-drying or alternatively by treatment with anhydrous acetone or ethanol followed by hot drying (40-45°C) under vacuum. The fraction of water-insoluble crosslinked HA in the product recovered is determined by washing the product in water followed by gravimetric measurement of the insoluble fraction. The percentage ratio between the mass of insoluble sample recovered and the mass of HA subjected to the reaction represents the reaction yield.

The advantages of the method claimed over the prior art are: a) that it produces a crosslinked "shape-memory" product which, after hydration, has the same geometry as the polysaccharide matrix reacted; b) that it maintains greater structural integrity of the polysaccharide chain, because the hydrolysis processes attributable to the basic reaction medium are more limited; c) the possibility of operating with very small reaction volumes; d) the possibility of easily modulating the crosslinking process; e) obtaining highly biocompatible crosslinked matrices with greater chemical stability at the implantation sites, which are not obtainable with the protocols currently developed for crosslinking with polyfunctional epoxides in a basic medium.

The examples below describe the invention in more detail.

EXAMPLE 1 - Effect of the amount of water on the crosslinking reaction

100 mL of reaction mixture containing different amounts of water and acetone (6.35, 16.35 and 33.05% v/v of water), which represent non-solvent systems for HA, wherein the organic base TMBAH (benzyl trimethyl ammonium hydroxide, 40% w/w ≡ 2.5 M aqueous solution) 10 mL (25 mmols) and the crosslinking agent BDDE 0.68 mL are dissolved (molar ratio BDDE/HA OH groups = 3.5%), is added to 10g of powdered HA (particles with dimensions between 0.2 and 80 μm); in view of the bi-functional nature of BDDE there is a theoretical possibility of engaging 7% of the hydroxyl moieties available on HA in the ether bond, namely an equivalent ratio of 7%. The reaction mixture to be added to the powdered HA is in the form of a homogeneous system with water percentages of 16.35 and 33.05% (v/v); however, with a water percentage of 6.35% (corresponding to the amount of water introduced with the organic base), the mixture is biphasic. After the HA is added, the reaction mixture, which presents as a paste-like consistency, is reacted for 3 h at 50°C under vigorous mixing. The reaction is stopped by adding concentrated $H_3PO_4$ (14.8 M) until neutralisation. The reaction mixture is purified as specified below: a) the liquid phase containing the soluble contaminants is removed; b) 4 washings are performed with 200 mL of a 53.5/46.5 v/v ethanol/water solution saturated with NaCl; c) 1 wash is performed with 200 mL of a 50/50 ethanol/water solution; d) pure ethanol is added until an ethanol/water ratio of 80/20 v/v is reached, and the liquid phase is removed; e) 10 washings are performed with 100 mL of an 80/20 ethanol/water solution); f) the crosslinked HA powder is treated with anhydrous ethanol (4 washings with 100 mL of anhydrous ethanol) and dried under vacuum at 40°C. The liquid phase is removed throughout the procedure by filtration through a porous ceramic membrane (pore size: 20 μm). Table 2 shows the composition of the different reaction mixtures and the reaction yields in insoluble crosslinked HA.

A particulate is obtained in the reaction with the same dimensional ratios as the HA used, with percentage yields compared with HA of 0.00 at 6.35 v/v of water, 43.4 ± 5.5 at 16.35 v/v of water and 79.4 ± 4.9 (%) at 33.05% v/v of water. The HA obtained using 33.05% v/v of water, when hydrated, appears as a continuous gel, whereas the HA crosslinked with 16.35% v/v of water, when hydrated, appears as a gelled particulate with a swelling factor of about 235 mL/g in water and 90 mL/g in saline. The differences in the experimental conditions clearly appear from a comparison between Table 2 and Table 1 relating to known methods, especially as regards the amount of water used.

Table 2 - Reaction mixture with variable amounts of water (including the water present in the TMBAH) and reaction yields as a % of crosslinked shape-memory water-insoluble HA.

| Sample (% $H_2O$)* | | HA (g) | Acetone (mL) | $H_2O$ (mL) | TMBAH** (mL solution/mmol TMBAH/mL$H_2O$ ) | BDDE (mL) | Insoluble° (%) |
|---|---|---|---|---|---|---|---|
| 1 | 6,35% | 10 | 89,32 | 0,0 | 10/25/6,35 | 0,68 | 0,0°° |
| 2 | 16,35% | 10 | 79,32 | 10,0 | 10/25/6,35 | 0,68 | 43,4 ± 5,5 |
| 3 | 33,05% | 10 | 62,62 | 26,7 | 10/25/6,35 | 0,68 | 79,4 ± 4,9 |

* total % of $H_2O$ in the reaction mixture, including that present in TMBAH; ** 40% w/w ≡ 2.5 M aqueous solution; ° % of crosslinked shape-memory HA; °° the yield is 0 because the optionally crosslinked product dissolves in the washing solutions during purification.

EXAMPLE 2 - Effect of time on crosslinking reaction

Six reaction mixtures are prepared, each formed by 10 g of powdered HA (particles with dimensions between 0.2 and 80 μm, to which 100 mL of reaction mixture is added, consisting of: water 10 mL, acetone 79.32 mL, TMBAH (benzyl trimethyl ammonium hydroxide, 40% w/w ≡ 2.5 M aqueous solution) 10 mL (25 mmols) and BDDE 0.68 mL (ratio in BDDE/HA equivalents = 7%). The HA is not solubilised in the reaction mixture, and the system has a paste-like consistency. The various samples are reacted at 50°C for times ranging between 0.5 and 6 h under energetic mixing. The reaction is stopped from time to time by adding 14.8 M $H_3PO_4$ until neutralisation. The various reaction mixtures are purified as described below: a) the liquid phase containing the soluble contaminants is removed; b) 4 washings are performed with 200 mL of a 53.5/46.5 v/v acetone/water solution saturated with NaCl; c) 1 wash is performed with 200 mL of a 50/50 acetone/water solution; d) pure acetone is added until an acetone/water ratio of 80/20 v/v is reached, and the liquid phase is removed; e) 10 washings are performed with 100 mL of an 80/20 acetone/water solution); f) the crosslinked HA powder is treated with anhydrous acetone (4 washings with 100 mL of anhydrous acetone) and dried under vacuum at 40°C. The reaction yields, defined as the % of crosslinked water-insoluble shape-memory HA obtained compared with the HA used in the reaction, are 0.00 at 0.5 h, 7.01 ± 1.2 at 1.5 h, 43.44 ± 5.5 at 3 h, 53.6 ± 6.6 at 5 h,

58.9 $\pm$ 5.9 at 7 h and 62.3 $\pm$ 4.4 at 16 h. In all cases a particulate is obtained with the same dimensional characteristics as the HA used in the reaction, which swells in water, giving rise to gel particles with an elastic consistency.

EXAMPLE 3 - Effect of temperature on the crosslinking reaction

5 reaction mixtures are prepared, each formed by 10 g of powdered HA (particles with dimensions between 0.2 and 80 $\mu$m), to which 100 mL of reaction mixture is added, consisting of: water 10 mL, acetone 79.32 mL, TMBAH (benzyl trimethyl ammonium hydroxide, 40% w/w $\equiv$ 2.5 M aqueous solution) 10 mL (25 mmols) and BDDE 0.68 mL (ratio in BDDE/HA equivalents = 7%). The HA is not solubilised in the reaction mixture, and the system has a paste-like consistency. The various samples are reacted at different temperatures (30, 40, 50, 60 and 70°C) for 3 h under vigorous mixing. The reaction is stopped from time to time by adding 14.8 M $H_3PO_4$ until neutralisation. The various reaction mixtures are purified as described in example 1. The reaction yields, defined as the % of crosslinked water-insoluble HA obtained compared with the HA used in the reaction, are 1.0 at 30°C, 28.0 $\pm$ 4.1 at 40°C, 43.4 $\pm$ 5.5 at 50°C, 66.0 $\pm$ 6.2 at 60° and 47.3 $\pm$ 7.3 at 70°C. In all cases a particulate is obtained with the same dimensional characteristics as the HA used in the reaction, which swells in water, giving rise to gel particles with an elastic consistency.

EXAMPLE 4 - Effect of concentration of base on the crosslinking reaction

6 reaction mixtures are prepared as shown in table 3, each formed by 10 g of powdered HA (particles with dimensions between 0.2 and 80 $\mu$m), to which 100 mL of reaction mixture is added, consisting of: acetone, water, BDDE 0.68 mL (ratio in BDDE/HA equivalents = 7%) and different amounts of TMBAH (benzyl trimethyl ammonium hydroxide, 40% w/w $\equiv$ 2.5 M aqueous solution) ranging from 0.025 to 0.64 M; the calculation of the amount of water at 16.35% takes account of the water present in the TMBAH. At TMBAH concentrations exceeding 0.3 M (30 mmol TMBAH in the reaction volume), the reaction mixture is biphasic.

Table 3 - Reaction mixture with variable amounts of TMBAH and reaction yields as a % of crosslinked water-insoluble shape-memory HA.

| Sample (H$_2$O %)* | | HA (g) | Acetone (mL) | H$_2$O (mL) | TMBAH* (mL added/mmol TMBAH in reaction/mL H$_2$O in base) | | | BDDE (mL) | Insoluble° (%) |
|---|---|---|---|---|---|---|---|---|---|
| 1 | 16,35 | 10 | 82,61 | 15,71 | 1,0 | 2,5 | 0,64 | 0,68 | 0,0°° |
| 2 | 16,35 | 10 | 81,15 | 13,17 | 5,0 | 12,5 | 3,18 | 0,68 | 18,2±1,0 |
| 3 | 16,35 | 10 | 79,32 | 10,00 | 10,0 | 25,0 | 6,35 | 0,68 | 43,4±5,5 |
| 4 | 16,35 | 10 | 78,60 | 8,72 | 12,0 | 30,0 | 7,63 | 0,68 | 54,8±1,8 |
| 5 | 16,35 | 10 | 75,69 | 3,63 | 20,0 | 50,0 | 12,72 | 0,68 | 66,9±1,2 |
| 6 | 16,35 | 10 | 73,65 | 0,07 | 25,6 | 64,0 | 16,28 | 0,68 | 64,6±3,2 |

* total % of H$_2$O in the reaction mixture, including that present in TMBAH; ** 40% w/w $\equiv$ 2.5 M aqueous solution; ° % of crosslinked shape-memory HA; °° the yield is 0 because although the product is crosslinked, it dissolves in the washing solutions during purification.

The HA is not solubilised in the reaction mixture, and the system has a paste-like consistency. The various samples are reacted at 50°C for 3 h under energetic mixing. The reaction is stopped by adding 14.8 M $H_3PO_4$ until neutralisation. The various reaction mixtures are purified as described in example 1. The reaction yields, defined as the % of crosslinked water-insoluble HA obtained compared with the HA used in the reaction, are 0.00 with 0.025 M TMBAH, 18.2 $\pm$ 2.8 with 0.125 M TMBAH, 43.4 $\pm$ 5.5 with 0.250 M TMBAH, 54.8 $\pm$ 1.8 with 0.300 M TMBAH, 66.9 $\pm$ 1.2 with 0.500 M TMBAH, and 64.6 $\pm$ 3.2 with 0.640 M TMBAH. In all cases a particulate is obtained with the same dimensional characteristics as the HA used in the reaction, which swells in water, giving rise to gel particles with an elastic consistency.

EXAMPLE 5 - Effect of amount of BDDE on the crosslinking reaction

4 reaction mixtures are prepared as shown in table 4, each formed by 10 g of powdered HA, particles with dimensions between 0.2 and 80 $\mu$m, to which 100 mL of reaction mixture is added, consisting of: water, acetone, TMBAH (benzyl trimethyl ammonium hydroxide, 40% w/w $\equiv$ 2.5 M aqueous solution) 10 mL (25 mmols) and different amounts of BDDE, namely 0.34, 0.68, 1.36 and 2.91 mL (ratio in BDDE/HA equivalents 3, 5, 7, 14 and 30% respectively). The HA is not solubilised in the reaction mixture, and the system has a paste-like consistency. The various samples are reacted at 50°C for 3 h under vigorous mixing.

Table 4 - Reaction mixture with variable amounts of TMBAH and reaction yields as a % of crosslinked water-insoluble shape-memory HA.

| Sample (% $H_2O$)* | | HA (g) | Acetone (mL) | $H_2O$ (mL) | TMBAH** (mLsol./mLH$_2$O° ) | BDDE (mL - %°) | | Insoluble°° (%) |
|---|---|---|---|---|---|---|---|---|
| 1 | 16,35% | 10 | 79,66 | 10,0 | 10/6,35 | 0,34 | 3,5 | 0,0^ |
| 2 | 16,35% | 10 | 79,32 | 10,0 | 10/6,35 | 0,68 | 7,0 | 43,4 ± 5,5 |
| 3 | 16,35% | 10 | 78,63 | 10,0 | 10/6,35 | 1,36 | 14,0 | 60,8 ± 7,2 |
| 4 | 16,35% | 10 | 77,09 | 10,0 | 10/6,35 | 2,91 | 30,0 | 74,5 ± 3,4 |

* total % of $H_2O$ in the reaction mixture, including that present in TMBAH; ** 40% w/w ≡ 2.5 M aqueous solution; ° amount of water contained in 10 mL of TMBAH; °ratio in BDDE/HA equivalents; °° % of crosslinked shape-memory HA; ^ the yield is 0 because although the product is crosslinked, it dissolves in the washing solutions during purification.

The reaction is stopped from time to time by adding 14.8 M $H_3PO_4$ until neutralisation. The various reaction mixtures are purified as described in example 1. The reaction yields, defined as the % of crosslinked water-insoluble HA obtained compared with the HA used in the reaction, are 0.0 with 0.34 mL of BDDE, 43.4 ± 5.5 with 0.68 mL of BDDE, 60.8 ± 7.2 with 1.36 mL of BDDE, and 74.5 ± 3.4 with 2.91 mL of BDDE. In all cases a particulate is obtained with the same dimensional characteristics as the HA used in the reaction, which swells in water, giving rise to gel particles with an elastic consistency.

EXAMPLE 6 - Swelling of HA-based shape-memory materials with different degrees of crosslinking in different types of aqueous systems.

The degree of swelling in water and saline solution under steady state conditions at the temperature of 37°C is determined for samples of crosslinked HA with a ratio of 7%, 11% and 14% in BDDE/HA equivalents.

3 reaction mixtures are prepared as shown in table 5, each formed by 10 g of powdered HA (particles with dimensions between 0.2 and 80 $\mu$m), to which 100 mL of reaction mixture is added, consisting of: water, acetone, TMBAH (benzyl trimethyl ammonium hydroxide, 40% w/w ≡ 2.5 M aqueous solution) 10 mL (25 mmols) and different amounts of BDDE, namely 0.68, 1.07 and 1.36 mL respectively (ratio in BDDE/HA equivalents 7, 11 and 14% respectively). The HA is not solubilised in the reaction mixture, and the system has a paste-like consistency. The various samples are reacted at 60°C for 3 h under vigorous mixing.

The reaction is stopped from time to time by adding 14.8 M $H_3PO_4$ until neutralisation. The various reaction mixtures are purified as described in example 1. The reaction yields, defined as the % of crosslinked water-insoluble HA obtained compared with the HA used in the reaction, are 65.7 ± 5.2 with 0.68 mL of BDDE, 70.8 ± 4.2 with 1.07 mL of BDDE, and 74.5 ± 5.3 with 1.36 mL of BDDE. In all cases a particulate is obtained with the same dimensional characteristics as the HA used in the reaction.

The samples prepared are resuspended in bidistilled water or saline at the concentration of 5 mg/mL. The suspensions are autoclaved (12 min, 120°C) in a graduated cylinder and left to swell for 12 h. The degree of swelling is determined by measuring the volume of gel in the cylinder, and expressed as mL of gel/g of crosslinked insoluble HA. All the measurements are performed in triplicate.

The swelling in water is 264 ± 13 mL/g for the sample with a ratio of 7% in BDDE/HA equivalents, 160 ± 11 mL/g for the sample with a ratio of 11% in BDDE/HA equivalents, and 115 ± 13 mL/g for the sample with a ratio of 14% in BDDE/HA equivalents.

Table 5 - Swelling degree values of crosslinked shape-memory HA samples with different amounts of BDDE. 100 mL of a reaction mixture consisting of $H_2O$ (17.65 mL), TMBAH (benzyl trimethyl ammonium hydroxide, 40% w/w $\equiv$ 2.5 M aqueous solution) 10 mL, corresponding to 25 mmols of TMBAH and 6.35 mL of $H_2O$, is added to 10g of powdered HA (particles with dimensions between 0.2 and 80 $\mu$m), in addition to BDDE and acetone as indicated in the table.

| Sample (% $H_{24}O$)* | Acetone (mL) | BDDE | | Insoluble° (%) | Swelling degree (mL/g) | |
|---|---|---|---|---|---|---|
| | | %** | mL | | water | saline |
| 1 | 71,67 | 7,0 | 0,68 | 65,7 $\pm$ 5,2 | 264 $\pm$ 13 | 110 $\pm$ 5 |
| 2 | 71,28 | 11,0 | 1,07 | 70,8 $\pm$ 4,2 | 160 $\pm$ 11 | 81 $\pm$ 5 |
| 3 | 70,99 | 14,0 | 1,36 | 74,5 $\pm$ 5,3 | 115 $\pm$ 13 | 75 $\pm$ 2 |
| * total % of $H_2O$ in the reaction mixture, including that present in TMBAH; **ratio in BDDE/HA equivalents; ° % of crosslinked shape-memory HA. | | | | | | |

The % swelling in saline for the sample is 110 $\pm$ 5 mL/g with a ratio of 7% in BDDE/HA equivalents, 81 $\pm$ 5 mL/g for the sample with a ratio of 11% in BDDE/HA equivalents, and 75 $\pm$ 2 mL/g for the sample with a ratio of 14% in BDDE/HA equivalents. As expected, the degree of swelling is greater in water than in saline, and an increase in the percentage of crosslinking agent reduces the amount of water absorbed per gram of sample, because the increased degree of crosslinking entails lower deformability of the material, and consequently lower porosity.

EXAMPLE 7 - Preparation of HA crosslinked with polyethylene glycol diglycidyl ether (PEGDGE)

2 reaction mixtures are prepared as shown in table 6, each formed by 10 g of powdered HA (particles with dimensions between 0.2 and 80 $\mu$m), to which 100 mL of reaction mixture is added, consisting of: water, acetone, TMBAH (benzyl trimethyl ammonium hydroxide, 40% w/w $\equiv$ 2.5 Mw aqueous solution) 10 mL (25 mmols) and different amounts of PEGDGE (Sigma Aldrich, Milan, Italy; product no. 475696, average $M_n$ = 526Da, d=1.14 g/mL), namely 2.0 and 3.1 mL (ratio in PEGDGE/HA equivalents: 9 and 14% and respectively). The HA is not solubilised in the reaction mixture, and the system has a paste-like consistency. The various samples are reacted at 60°C for 3 h under vigorous mixing.

Table 6 - Reaction mixture with variable amounts of PEGDGE and reaction yields as a % of crosslinked water-insoluble shape-memory HA

| Sample (% $H_2O$)* | | HA (g) | Acetone (mL) | $H_2O$ (mL) | TMBAH** (mLsol./mLH$_2$O°) | PEGDGE (mL - %°) | | Insoluble°° (%) |
|---|---|---|---|---|---|---|---|---|
| 1 | 24% | 10 | 70,35 | 17,65 | 10/6,35 | 2,0 | 9 | 61,0 $\pm$ 3,5 |
| 2 | 24% | 10 | 69,25 | 17,65 | 10/6,35 | 3,1 | 14 | 74,0 $\pm$ 4,5 |
| * total % of $H_2O$ in the reaction mixture, including that present in TMBAH; ** 40% w/w $\equiv$ 2.5 M aqueous solution; ° amount of water contained in 10 mL of TMBAH; °ratio in PEGDGE/HA equivalents; °° % of crosslinked shape-memory HA. | | | | | | | | |

The reaction is stopped from time to time by adding 14.8 M $H_3PO_4$ until neutralisation. The various reaction mixtures are purified as described in example 1. The reaction yields, defined as the % of crosslinked water-insoluble HA obtained compared with the HA used in the reaction, are 61.0 $\pm$ 3.5 with 0.20 mL of PEGDGE, and 74.5 $\pm$ 4.5 with 0.31 mL of PEGDGE. In all cases a particulate is obtained with the same dimensional characteristics as the HA used in the reaction.

EXAMPLE 8 - Evaluation of stability to hyaluronidase of HA-based shape-memory materials with different degrees of crosslinking

The stability of the shape-memory materials based on HA with different degrees of crosslinking, described in example 6, to the degradative action of bovine testicular hyaluronidase (BTH), was determined. BHT is an endoglucan hydrolase, whose optimum pH for hydrolase activity is about 4-5. The study was conducted by comparison with Restylane®, a product based on HA crosslinked with BDDE, which is widely used as an intradermal filler for aesthetic medicine treatments. The samples are suspended at the concentration of 4 mg/mL in phosphate buffer pH 7.4, autoclaved (12 min at 120°C) and incubated at 37°C under stirring (1,000 rpm) with 50 U/mL of BTH (in the case of Restylane, the product is directly diluted in phosphate buffer and incubated with BTH). At the established times, the samples are heated to 100°C for 10 min to stop the enzymatic activity. The samples are then filtered through an 0.45 $\mu$m filter and the filtrate (containing the soluble fraction of the sample) is tested for HA content with the carbazole test (T. Bitter, H.M. Muir, Anal. Biochem. 1962, 330-334).

The degradation is established by monitoring the increase in the soluble fraction over time.

$$\% \text{ soluble HA} = \frac{\text{HA mass in permeate (g)}}{\text{HA mass incubated (g)}} \times 100$$

Table 7 shows the data obtained. The products analysed present substantial differences in degradation times. In particular, the degradation rate of the products described in example 6 declines as the degree of crosslinking increases: the sample crosslinked with 7% BDDE solubilises completely at incubation times between 6 and 24 h; the product crosslinked with 11% BDDE reaches complete solubilisation after incubation times 10 times longer; when the product crosslinked with 11% BDDE is completely solubilised, the soluble fraction for the product crosslinked with 14% BDDE amounts to about 50% of the total. Under the same experimental conditions, the commercial product Restylane solubilises completely in less than 3 h.

Table 7 - Stability *in vitro* at 37°C of samples of crosslinked shape-memory HA with different amounts of BDDE prepared according to example 6, and of a commercial sample of *Restylane,* to hydrolysis catalysed by bovine hyaluronidase (BTH 50 U/mL) [®].

| Sample (% BDDE)* | % soluble after incubation with BTH 50 U/mL | | | | | | | |
|---|---|---|---|---|---|---|---|---|
| | **0h** | **1h** | **3h** | **6h** | **24h** | **3 days** | **10 days** | **17 days** |
| 1 (7) | 3,7 ± 0.5 | 53,8 ± 5.3 | 83,7 ± 2,8 | 90,7 ±1.8 | 100 | | | |
| 2(11) | 4.0 ± 0,1 | | 25,0 ± 1,0 | | 69,0 ± 0,3 | 77 ± 7 | 97,0 ± 0,5 | |
| 3 (14) | 0,1 ± 0,0 | | 10,2 ± 1,1 | | 18,4 ± 0,8 | 29,8 ±4,1 | 51,8 ± 1,8 | 61,0 ± 4,0 |
| *Restylane*[®] | 33.0 ± 5.0 | | 100 | | | | | |
| °ratio in BDDE/HA equivalents. | | | | | | | | |

EXAMPLE 9 - Extrudability of shape-memory particulates based on crosslinked HA

In view of the possible use of particulates based on crosslinked shape-memory HA in the field of aesthetic medicine as fillers to be microinjected under the skin, the extrudability of suspensions in saline solution (0.9% w/v NaCl) of crosslinked HA samples with a ratio of 7%, 11% and 14% in BDDE/HA equivalents, prepared as described in example 6, has been evaluated. The crosslinked samples are left to swell in saline solution at the concentrations of 20, 25 and 30 mg/mL. The suspensions are kept under stirring (1,000 rpm) for 2-3 h. All the samples swelled until they became gels with an elasticity that decreases as the concentration of the suspensions and the degree of crosslinking increase. 2 mL glass syringes were filled with the hydrated suspensions, and then underwent a sterilisation cycle for 12 min at 120°C. The sterilised gels were extruded from the syringes through 27 and 30 gauge needles, corresponding to internal diameters of 0.45 and 0.30 mm respectively (these dimensions correspond to those of the needles used to inject the HA-based fillers currently on the market). The sample of crosslinked HA with a ratio of 7% in BDDE/HA equivalents produced suspensions extrudable through a 30 gauge needle at all the concentrations tested. For the samples crosslinked with ratios of 11% and 14% in BDDE/HA equivalents, the most concentrated suspensions (30 mg/mL) proved to be extrudable through gauge 27 needles, and those at lower concentrations through gauge 30 needles. The samples were deemed extrudable by applying pressures compatible with clinical application. The extrudability of the gels depends on the particle size in the hydrated state and on their elasticity. In particular, extrudability improves with a reduction in concentration and the size of the hydrated particles, and an increase in their elasticity. The samples of crosslinked HA described in example 6 present (in the dry state) the same shape and size characteristics (0.2-80 $\mu$m) as the HA used in the crosslinking reaction. Considering the swelling degree values in saline solution reported in table 5 (110 $\pm$ 5 mL/g for the 7% crosslinked sample in BDDE/HA equivalents, 81 $\pm$ 5 mL/g for the 11% crosslinked sample in BDDE/HA equivalents, and 75 $\pm$ 2 mL/g for the 14% crosslinked sample in BDDE/HA equivalents), after hydration the particle size increases 110- to 75-fold on average compared with the dry state, changing from the less crosslinked to the more crosslinked product and reaching dimensions ranging from 15 $\mu$m to 8.8 mm. The easy extrudability through 27-30 gauge needles (inner diameter 0.45-0.30 mm) therefore demonstrates the great elasticity of the crosslinked shape-memory HA particulates after hydration, an important characteristic not only for the injectability of the product but also for the success of the filler at the site of implantation in aesthetic terms.

EXAMPLE 10 - Preparation of a crosslinked shape-memory HA sponge

Crosslinked HA sponges are prepared from high-molecular-weight HA (HHA, Mw 1.5kDa) and low-molecular-weight HA (LHA, Mw 220kDa).

Aqueous solutions of 4% w/w HA in the case of HHA and 12.5% w/w in the case of LHA are poured into petri dishes to a liquid thickness of 0.5 cm, and freeze-dried. In both cases a material is obtained which, when observed under the scanning electron microscope, presents as a porous structure with a high degree of pore interconnection. The material thus obtained solubilises instantly when placed in water. To obtain a material that retains its original shape in water, the freeze-dried sponge is subjected to crosslinking processes with BDDE in a non-solvent system. In particular, 3 reaction mixtures are prepared for both materials based on HHA and LHA, each consisting of 10g of HA sponge to which 100 mL of reaction mixture is added, consisting of: water 10 mL, TMBAH (benzyl trimethyl ammonium hydroxide, 40% w/w $\equiv$ 2.5 M aqueous solution) 10 mL (25 mmols) and variable amounts of BDDE and acetone as indicated in table 8. In particular, the amount of BDDE used corresponds to ratios of 7, 14 and 30% in BDDE/HA equivalents. The material, imbibed and entirely covered by the reaction mixture, is reacted at 60°C for 3 h under stirring; the reaction is stopped by adding 14.8 M $H_3PO_4$ until neutralisation. The crosslinked product is purified as described in example 1. All the materials obtained retain their original three-dimensional shape, but in water, as the degree of crosslinking increases, they present growing resistance and swell to a decreasing extent.

Table 8 - Reaction mixture with variable amounts of BDDE

| Sample (% $H_2O$)* | | HA** (g) | Acetone (mL) | $H_2O$ (mL) | TMBAH° (mL/mLH$_2$O °°) | BDDE (mL - %^) | |
|---|---|---|---|---|---|---|---|
| 1 | 16,35% | 10 | 79,32 | 10,0 | 10/6,35 | 0,68 | 7,0 |
| 2 | 16,35% | 10 | 78,63 | 10,0 | 10/6,35 | 1,36 | 14,0 |
| 3 | 16,35% | 10 | 77,09 | 10,0 | 10/6,35 | 2,91 | 30,0 |
| * total % of $H_2O$ in the reaction mixture, including that present in TMBAH; ** Sponge based on HHA or LHA; ° 40% w/w $\equiv$ 2.5 M aqueous solution; °° amount of water contained in 10 mL of TMBAH; ^ ratio in BDDE/HA equivalents. | | | | | | | |

EXAMPLE 11 - Preparation of crosslinked chitosan

100 mL of reaction mixture, containing 24% v/v water in acetone, which represents a non-solvent system for chitosan wherein the organic base TMBAH (benzyl trimethyl ammonium hydroxide, 40% w/w $\equiv$ 2.5 M aqueous solution) 10 mL (25 mmols) and the crosslinking agent, BDDE 2.26 mL (ratio in BDDE/OH/$NH_2$ group equivalents of chitosan = 14%) are dissolved, is added to 10g of powdered chitosan (low-molecular-weight chitosan, Sigma Aldrich, Milan, Italy; product no. 448869, deacetylation = 75%, viscosity 20-300 cps c = 1% w/v in acetic acid, 1% v/v in water). A reaction mixture to which the crosslinking agent is not added is used as control system.

The chitosan is not solubilised in the reaction mixture, and the system has a paste-like consistency. The sample is reacted at 60°C for 3 h under vigorous mixing.

The reaction is stopped by adding 14.8 M $H_3PO_4$ until neutralisation. The two reaction mixtures, with and without crosslinking agent, are purified as reported in example 1. A particulate with the same dimensional characteristics as the chitosan used in the reaction is obtained, with percentage yields compared with the chitosan used of 95 and 93% w/w respectively for the reaction mixture with and without crosslinking agent. To evaluate the yield of insoluble crosslinked product, the two powders are suspended in a 1% v/v aqueous solution of acetic acid which is a solvent system for chitosan, left under stirring for 5 h and centrifuged. The sediment is washed twice with water and dried by freeze-drying. The lyophilisate represents 80% of the reaction product for the mixture with BDDE, while the powder of the reaction mixture without BDDE solubilises completely in 1% v/v water/acetic acid.

EXAMPLE 12 - Crosslinked HA functionalised with choline

Crosslinked shape-memory HA containing choline chloride residues was produced.

The reaction in the presence of choline chloride was conducted according to two procedures. For the first one, 100 mL of reaction mixture consisting of water, acetone, TMBAH (benzyl trimethyl ammonium hydroxide, 40% w/w $\equiv$ 2.5 M aqueous solution) 10 mL (25 mmols), a amount of BDDE amounting to 1.36 mL (ratio in BDDE/HA equivalents 14%) and choline chloride (C7017, Sigma Aldrich, Italy) 1.94 g (ratio in choline/HA equivalents 14%) is added to 10 g of powdered HA (particles with dimensions between 0.2 and 80 $\mu$m). The HA is not solubilised in the reaction mixture, and the system has a paste-like consistency. The sample is reacted at 60°C for 3 h under vigorous mixing.

For the second procedure, 100 mL of reaction mixture, consisting of water, acetone, TMBAH (benzyl trimethyl ammonium hydroxide, 40% w/w $\equiv$ 2.5 M aqueous solution) 10 mL (25 mmols) and 1.36 mL of BDDE (ratio in BDDE/HA equivalents 14%) is added to 10g of powdered HA (particles with dimensions between 0.2 and 80 $\mu$m). The HA is not solubilised in the reaction mixture, and the system has a paste-like consistency. The sample is reacted at 60°C for 3 h under vigorous mixing. After 3 h, a further 100 mL of reaction mixture is added, consisting of: water, acetone, TMBAH (benzyl trimethyl ammonium hydroxide, 40% w/w $\equiv$ 2.5 M aqueous solution) 10 mL (25 mmols) and BDDE 1.36 mL (ratio in BDDE/HA equivalents 14%) and choline chloride (C7017, Sigma Aldrich, Italy) 1.94 g (ratio in choline/HA equivalents 14%). The HA is not solubilised in the reaction mixture, and the system has a paste-like consistency. The sample is reacted at 60°C for a further 1.5 h under vigorous mixing.

The reaction is stopped from time to time by adding 14.8 M $H_3PO_4$ until neutralisation. The various reaction mixtures are purified as described in example 1. Particulates are obtained with the same dimensional characteristics as the HA used in the reaction. The reaction yields, defined as the % of crosslinked water-insoluble HA obtained compared with the HA used in the reaction, are 29 $\pm$ 2 and 71 $\pm$ 3 respectively).

For both products, the swelling degree was evaluated as described in example 6. The swelling degree for the sample obtained by the first procedure is 270 $\pm$ 16 mL/g in water and 94 $\pm$ 8 mL/g in saline. The swelling degree for the sample obtained by the second procedure is 139 $\pm$ 11 mL/g in water and 81 $\pm$ 4 mL/g in saline.

Table 9 - Yield and swelling degree values of samples of shape-memory HA crosslinked with BDDE in the presence of choline chloride. For sample 1, 100 mL of a reaction mixture consisting of: $H_2O$ (17.65 mL), TMBAH (benzyl trimethyl ammonium hydroxide, 40% w/w $\equiv$ 2.5M aqueous solution) 10 mL, corresponding to 25 mmols of TMBAH and 6.35 mL of $H_2O$, is added to 10 g of powdered HA (particles with dimensions between 0.2 - 80 $\mu$m) in addition to BDDE, acetone and choline chloride, as reported in the table. For sample 2, 100 mL + 100 mL of reaction mixture containing $H_2O$ (17.65 mL), TMBAH (benzyl trimethyl ammonium hydroxide, 2.5 M $\equiv$ 40% w/w aqueous solution) 10 mL, corresponding to 25 mmols of TMBAH and 6.35 mL of $H_2O$, is added to 10g of powdered HA (particles with dimensions between 0.2 and 80 $\mu$m), in addition to BDDE, acetone and choline chloride as shown in the table.

| Sample (24% $H_2O$)* | Acetone (mL) | BDDE | | Choline chloride | | Insoluble° (%) | Swelling degree (mL/g) | |
|---|---|---|---|---|---|---|---|---|
| | | %** | mL | *** | g | | Water | Saline |
| 1 | 70,99 | 14,0 | 1,36 | 100 | 1,94 | 29 $\pm$ 2 | 270 $\pm$ 16 | 94 $\pm$ 8 |
| 2 | 70,99 +70,99 | 14,0 +14,0 | 1,36 +1,36 | 0 +100 | 0 +1,94 | 68 $\pm$ 3 | 139 $\pm$ 11 | 81 $\pm$ 4 |
| * total % of $H_2O$ in the reaction mixture, including that present in TMBAH; **ratio in BDDE/HA equivalents; ***ratio in choline/BDDE equivalents; ° % of crosslinked shape-memory HA. | | | | | | | | |

As expected on the basis of the competition between HA and choline in the reaction with BDDE, reaction 1 leads to a less crosslinked product than that obtained in the absence of choline (example 6): lower yield and higher swelling degree values. For reaction 2, the yield value is only comparable with that of the product obtained with 14% BDDE as described in example 6; however, the swelling degree values are higher, evidently due to the introduction of choline residues into the network.

The products described were tested for stability to the degradative action of bovine testicular hyaluronidase according to the procedure described in example 7. Table 10 shows the results obtained.

As expected, the products analysed differ in terms of degradation times. In particular, sample 1 is completely solubilised after 5 days' incubation. Under the same conditions, sample 2 is only 44 $\pm$ 3% soluble. Both samples proved less resistant to the enzymatic action of the product crosslinked with 14% BDDE in the absence of choline obtained as described in example 6. These results are consistent with the lower degree of crosslinking and higher degree of swelling (greater exposure to enzymatic action) of sample 1, and the higher degree of swelling of sample 2 compared with the sample crosslinked with 14% BDDE in the absence of choline.

Table 10 - Stability *in vitro* at 37°C of shape-memory HA samples crosslinked with BDDE in the presence of choline chloride to hydrolysis catalysed by bovine hyaluronidase (BTH 50 U/mL)

| Sample (24% H$_2$O)* | Acetone (mL) | BDDE | | Choline chloride | | % soluble after incubation with BTH 50 U/mL | |
| --- | --- | --- | --- | --- | --- | --- | --- |
| | | %** | mL | *** | g | 3 days | 5 days |
| 1 | 70,99 | 14,0 | 1,36 | 100 | 1,94 | 44 ± 1 | 0 |
| 2 | 70,99 + 70,99 | 14,0 + 14,0 | 1,36 + 1,36 | 0 + 100 | 0 + 1,94 | 57 ± 5 | 44 ± 3 |
| * total % of H$_2$O in the reaction mixture, including that present in TMBAH; **ratio in BDDE/HA equivalents; ***ratio in choline/BDDE equivalents. | | | | | | | |

EXAMPLE 13 - Evaluation of cytotoxicity of shape-memory materials based on crosslinked HA

The *in vitro* cytotoxicity tests are conducted according to the ISO 10993-5 standard (indirect test). In particular, a cell culture of murine fibroblasts NIH3T3 is incubated with culture medium conditioned with the samples to be analysed. The tests are performed in triplicate.

The evaluation is conducted on products based on crosslinked HA prepared as described in example 5 (powdered crosslinked HA using a ratio of 7, 14 and 30% in BDDE/HA equivalents) and on products based on crosslinked HA prepared as described in example 9 (HHA and LHA sponges crosslinked with a ratio of 30% in BDDE/HA equivalents).

Operating under sterile conditions, the conditioned media are obtained by placing the test materials in complete DMEM without phenol red (the volume of medium added is such that when swelling equilibrium is reached, the materials are suspended in 1 mL of medium/0.2 g of material) and incubating them at 37°C for 24 h. The culture medium used is Dulbecco's modified Eagle's medium (DMEM) containing glucose (4.5 g/L), sodium pyruvate (1 mM) and glutamine (2 mM) with the addition of 10% v/v foetal bovine serum (FBS), penicillin (100 U/mL), streptomycin (100 μg/mL), fungizone (2.5 μg/mL) and non-essential amino acids (1% v/v).

At the end of the incubation the culture medium is centrifuged and the supernatant represents the conditioned medium. Simultaneously, murine fibroblasts NIH3T3 are seeded at a density of 8.5x10$^4$cell/cm$^2$ in a 12-well multiwell cell culture plate and incubated in complete DMEM without phenol red at 37°C for 24 h. 24 h after seeding, the culture medium is replaced with 1 mL of medium conditioned with the test materials, and the plates are incubated at 37°C for 48 h. In the control wells, the medium is replaced with complete unconditioned DMEM.

After 24 and 48 h of incubation with conditioned medium, cell viability is evaluated qualitatively by observation under the optical microscope, and quantitatively with the MTT test (3-[4,5 dimethylthiazol-2-yl]-2,5 diphenyltetrazolium bromide) (Slater TF, Sawyer B, Strauli U., Biochim Biophys Acta 1963; 77, 383-393). In particular, the viability of the cells incubated with conditioned medium was quantified in percentage terms compared with that of the cells incubated with unconditioned medium (control).

Under the optical microscope, the cells incubated with all the conditioned media present their optimum morphology, indicating that there is no release of toxic substances from the materials.

Similarly, quantitative analysis with the MTT test confirms that the materials tested are not cytotoxic, because the cells incubated with conditioned medium with all the crosslinked HA samples presented a viability comparable with that of the control (80-100%).

EXAMPLE 14 - *In vivo* evaluation of crosslinked shape-memory HA

The cytocompatibility of crosslinked shape-memory HA sponges is evaluated *in vivo* using the mouse as model system.

A 12.5% w/w solution of LHA in water is poured into a petri dish until the thickness of the liquid is 0.5 cm; the solution is then freeze-dried. The freeze-dried material, which presents a porous structure with a high degree of pore interconnection when observed under the scanning electron microscope, is then crosslinked. In particular, 194 mL of reaction mixture consisting of water 19.4 mL, acetone 149.6 mL, TMBAH (benzyl trimethyl ammonium hydroxide, 40% w/w ≡ 2.5 M aqueous solution) 19.4 mL (48.5 mmols) and BDDE 5.64 mL (ratio in BDDE/HA equivalent = 58.2%) is added to 10 g of freeze-dried LHA. The HA is not solubilised in the reaction mixture, and the material is imbibed and entirely covered by the liquid phase. The sample is reacted at 60°C for 3 h under vigorous mixing; the reaction is stopped by adding 14.8 M H$_3$PO$_4$ until neutralisation. The crosslinked product is purified as described in example 1. The material obtained, which is insoluble in an aqueous medium, presents as an elastic hydrogel which, as demonstrated by observations under the scanning electron microscope and optical microscope, maintains the porous structure of the crosslinked material.

The next step is preparation of the construct, a scaffold loaded with human chondrocytes, to be implanted under the skin of immunocompromised mice.

The human chondrocytes are obtained from human cartilage removed during rhinoplasty operations. The piece of cartilage, stored under sterile conditions, is divided, again under sterile conditions, into small pieces which are enzymatically disintegrated by incubating the material under stirring for 12 h at 37°C in a PBS solution containing type I collagenase (3 mg/mL), dispase (4 mg/mL) and 5 $\mu$l/mL of a gentamicin solution (80 mg/mL). The suspension is then filtered through an 0.2 $\mu$m filter and the cell residue is washed with DMEM 10%FBS medium. The resulting solution is centrifuged at 1,500 rpm for 7 min, the supernatant is eliminated and the pellet is resuspended in DMEM 10%FBS. The chondrocytes thus obtained are seeded on a plate in DMEM 10%FBS containing DIFLUCAN at the concentration of 5 $\mu$L/mL and gentamicin 5 $\mu$l/mL.

For the preparation of the scaffold, the crosslinked shape-memory HA sponge is cut to the shape of discs, which have a diameter of about 10 mm when swelling equilibrium is reached. Said discs, still in the dry state, are sterilised in the autoclave (12 min at 120°C) and then loaded with human chondrocytes, leaving the hydrogel to swell *in vitro* with a cell suspension containing $2 \times 10^5$ cells until complete absorption. Constructs of Hyalofill™ (Fidia Advanced Biopolymers) and chondrocytes are prepared in the same way and used in the *in vivo* study as control. The constructs obtained are incubated for 48 h at 37°C and 5% $CO_2$ to allow the cells to adhere to the scaffold, and then implanted subcutaneously *in vivo* in the dorsal area of immunocompromised mice.

10 previously acclimatised animals are used in the study. In the dorsal area of five rats, two discs of crosslinked shape-memory HA are implanted on the right-hand side and two discs of the same material, loaded with human chondrocytes, on the left-hand side; a similar procedure is followed on the other five animals, implanting Hyalofill and Hyalofill loaded with human chondrocytes. 30 days after implantation the constructs are removed, if still present, and tissue samples are taken from the peri-implant area. This material undergoes immunohistochemical and histological tests. The Hyalofill was completely resorbed, whether implanted without chondrocytes or combined with chondrocytes, but in the case of the crosslinked shape-memory HA sponge, both the control and the scaffold loaded with human chondrocytes were still *in situ,* and were extensively vascularised. Histological analysis of the crosslinked HA construct loaded with human chondrocytes shows the presence of structurally well organised cartilage tissue.

## Claims

1. A process for the preparation of crosslinked polysaccharides selected from hyaluronic acid, chondroitin and chondroitin sulphate, which comprises cross-linking the polysaccharide in a non-solvent system for the polysaccharide consisting of mixtures of organic solvents and water in the presence of quaternary ammonium bases in concentrations from 0.025 to 1.3 M and of a polyfunctional epoxide, at a temperatures from 30 to 80°C for reaction times from 2 to 16 hours, said cross-linked polysaccharides holding their 3D shape during crosslinking.

2. A process according to claim 1 wherein the polysaccharide is hyaluronic acid.

3. A process according to claim 1 or 2 wherein the non-solvent system consists of mixtures wherein the volume ratio of organic solvent to water is $\geq 2$.

4. A process according to claim 3 wherein the organic solvents are water-miscible ketones, alcohols or ethers, preferably acetone.

5. A process according to one or more of claims 1-4 wherein the quaternary ammonium bases are selected from tetrabutyl ammonium hydroxide, tetrapropyl ammonium hydroxide, tetraethyl ammonium hydroxide, tetramethyl ammonium hydroxide, benzyl tributyl ammonium hydroxide, benzyl tripropyl ammonium hydroxide, benzyl triethyl ammonium hydroxide, benzyl trimethyl ammonium hydroxide, methyl tributyl ammonium hydroxide, methyl tripropyl ammonium hydroxide, methyl triethyl ammonium hydroxide, phenyl tributyl ammonium hydroxide, phenyl tripropyl ammonium hydroxide, phenyl triethyl ammonium hydroxide, phenyl trimethyl ammonium hydroxide, dodecyl trimethyl ammonium hydroxide, tetradecyl trimethyl ammonium hydroxide, hexadecyl trimethyl ammonium hydroxide, octadecyl trimethyl ammonium hydroxide and choline hydroxide.

6. A process according to claim 5 wherein the quaternary ammonium base is benzyl trimethyl ammonium hydroxide.

**7.** A process according to one or more of claims 1-6 wherein the polyfunctional epoxide is selected from 1,3-butadiene diepoxide, 1,2,7,8-diepoxyoctane, epoxide 1,5-hexadiene, ethylene glycol diglycidyl ether, 1,4-butanediol diglycidyl ether, 1,6-hexanediol diglycidyl ether, polyethylene glycol diglycidyl ether, polypropylene glycol diglycidyl ether, bisphenol A diglycidyl ether, preferably 1,4-butanediol diglycidyl ether, polyethylene glycol diglycidyl ether and polypropylene glycol diglycidyl ether.

**8.** A process according to claim 1 wherein the reaction is interrupted by addition of a concentrated acid to neutrality.

**9.** A process according to one or more of claims 1-8 wherein the crosslinked product is purified by removing the liquid phase and washing until disappearance of contaminants with a homogeneous water/organic solvent mixture, which does not increase the swelling of the solid phase.

**10.** A process according to claim 9 wherein the water/organic solvent mixture is saturated with sodium chloride in the first washings.

**11.** A process according to claim 9 or 10 wherein the organic solvent present in the washing mixture is ethanol or acetone.

**12.** A process according to claim 9 wherein the ratio of organic solvent to water is $\geq 2$.

**13.** A process according to one or more of claims 1-12 wherein the crosslinked product is obtained by drying the material after washing.

**14.** A process according to claim 13 wherein drying is obtained by exhaustive washing with a water-miscible anhydrous organic solvent and subsequent drying under vacuum at high temperature or by freeze-drying.

**15.** Materials obtained by the processes of claims 1 to 14.

## Patentansprüche

**1.** Verfahren zur Herstellung von vernetzten Polysacchariden ausgewählt von Hyaluronsäure, Chondroitin und Chondroitinsulfat, welches das Vernetzen der Polysaccharide in einem lösungsmittelfreien System für die Polysaccharide, bestehend aus Mischungen von organischen Lösungsmitteln und Wasser in der Gegenwart von quaternären Ammoniumbasen in einer Konzentration von 0.025 bis 1.3 M und einem polyfunktionalen Epoxid, bei Temperaturen von 30 bis 80°C für eine Reaktionszeit von 2 bis 16 Stunden umfasst, wobei besagte vernetzte Polysaccharide ihre 3D Form während des Vernetzens beibehalten.

**2.** Verfahren gemäß Anspruch 1, wobei das Polysaccharid Hyaluronsäure ist.

**3.** Verfahren gemäß Anspruch 1 oder 2, wobei das lösungsmittelfreie System aus Mischungen besteht, bei denen das Volumenverhältnis von organischem Lösungsmittel zu Wasser $\geq 2$ ist.

**4.** Verfahren gemäß Anspruch 3, wobei die organischen Lösungsmittel wassermischbare Ketone, Alkohole oder Ether, bevorzugt Aceton sind.

**5.** Verfahren gemäß einem oder mehreren der Ansprüche 1-4, wobei die quaternären Ammoniumbasen ausgewählt sind aus Tetrabutylammoniumhydroxid, Tetrapropylammoniumhydroxid, Tetraethylammoniumhydroxid, Tetramethylammoniumhydroxid, Benzyltributylammoniumhydroxid, Benzyltripropylammoniumhydroxid, Benzyltriethylammoniumhydroxid, Benzyltrimethylammoniumhydroxid, Methyltributylammoniumhydroxid, Methyltripropylammoniumhydroxid, Methyltriethylammoniumhydroxid, Phenyltributylammoniumhydroxid, Phenyltripropylammoniumhydroxid, Phenyltriethylammoniumhydroxid, Phenyltrimethylammoniumhydroxid, Dodecyltrimethylammoniumhydroxid, Tetradecyltrimethylammoniumhydroxid, Hexadecyltrimethylammoniumhydroxid, Octadecyltrimethylammoniumhydroxid und Cholinhydroxid.

**6.** Verfahren gemäß Anspruch 5, wobei die quaternäre Ammoniumbase Benzyltrimethylammoniumhydroxid ist.

**7.** Verfahren gemäß einem oder mehreren der Ansprüche 1-6, wobei das polyfunktionale Epoxid ausgewählt ist aus 1,3-Butadien-diepoxid, 1,2,7,8-Diepoxyoctan, Epoxid-1,5-hexadien, Ethylenglycol-diglycidylether, 1,4-Butandioldiglycidylether, 1,6-Hexandiol-diglycidylether, Polyethylenglycol-diglycidylether, Polypropylenglycol-diglycidylether, Bisphenol A-diglycidylether, bevorzugt 1,4-Butandiol-diglycidylether, Polyethylenglycol-diglycidylether und Polypropylenglycol-diglycidylether ist.

8. Verfahren gemäß Anspruch 1, wobei die Reaktion durch die Zugabe von einer konzentrierten Säure bis zu Neutralität unterbrochen wird.

9. Verfahren gemäß einem oder mehreren der Ansprüche 1-8, wobei das vernetzte Produkt, durch Entfernen der flüssigen Phase und Waschen mit einer homogenen Mischung aus Wasser/organischem Lösungsmittel bis zum Verschwinden von Verunreinigungen, gereinigt wird, was das Schwellen der festen Phase nicht erhöht.

10. Verfahren gemäß Anspruch 9, wobei die Mischung aus Wasser/organischem Lösungsmittel bei den ersten Wäschen mit Natriumchlorid gesättigt ist.

11. Verfahren gemäß Anspruch 9 oder 10, wobei das organische Lösungsmittel, welches in der Waschmischung anwesend ist, Ethanol oder Aceton ist.

12. Verfahren gemäß Anspruch 9, wobei das Verhältnis von organischem Lösungsmittel zu Wasser $\geq 2$ ist.

13. Verfahren gemäß einem oder mehreren der Ansprüche 1-12, wobei das vernetzte Produkt durch Trocken des Materials nach dem Waschen erhalten wird.

14. Verfahren gemäß Anspruch 13, wobei das Trocknen durch gründliches Waschen mit einem wassermischbaren wasserfreien organischem Lösungsmittel und anschließendem Trocknen unter Vakuum bei hohen Temperaturen oder durch Gefriertrocknen erhalten wird.

15. Materialien erhältlich durch das Verfahren gemäß den Ansprüchen 1 bis 14.

**Revendications**

1. Procédé pour la préparation de polysaccharides réticulés choisis parmi l'acide hyaluronique, la chondroïtine et le sulfate de chondroïtine, qui comprend la réticulation du polysaccharide dans un système de non-solvant pour le polysaccharide, constitué par des mélanges de solvants organiques et d'eau en présence de bases d'ammonium quaternaires dans des concentrations de 0,025 à 1,3 M et d'un époxyde polyfonctionnel à des températures de 30 à 80 °C pendant des durées de réaction de 2 à 16 heures, lesdits polysaccharides réticulés maintenant leur configuration en 3D au cours de la réticulation.

2. Procédé selon la revendication 1, dans lequel le polysaccharide est l'acide hyaluronique.

3. Procédé selon la revendication 1 ou 2, dans lequel le système de non-solvant est constitué par des mélanges dans lesquels le rapport volumique du solvant organique à l'eau est $\geq 2$.

4. Procédé selon la revendication 3, dans lequel les solvants organiques sont des cétones, des alcools ou des éthers miscibles à l'eau, de préférence l'acétone.

5. Procédé selon une ou plusieurs des revendications 1 à 4, dans lequel les bases d'ammonium quaternaires sont choisies parmi l'hydroxyde de tétrabutyl ammonium, l'hydroxyde de tétrapropyl ammonium, l'hydroxyde de tétraéthyl ammonium, l'hydroxyde de tétraméthyl ammonium, l'hydroxyde de benzyl tributyl ammonium, l'hydroxyde de benzyl tripropyl ammonium, l'hydroxyde de benzyl triéthyl ammonium, l'hydroxyde de benzyl triméthyl ammonium, l'hydroxyde de méthyl tributyl ammonium, l'hydroxyde de méthyl tripropyl ammonium, l'hydroxyde de méthyl triéthyl ammonium, l'hydroxyde de phényl tributyl ammonium, l'hydroxyde de phényl tripropyl ammonium, l'hydroxyde de phényl triéthyl ammonium, l'hydroxyde de phényl triméthyl ammonium, l'hydroxyde de dodécyl triméthyl ammonium, l'hydroxyde de tétradécyl triméthyl ammonium, l'hydroxyde d'hexadécyl triméthyl ammonium, l'hydroxyde d'octadécyl triméthyl ammonium et l'hydroxyde de choline.

6. Procédé selon la revendication 5, dans lequel la base d'ammonium quaternaire est l'hydroxyde de benzyl triméthyl ammonium.

**7.** Procédé selon une ou plusieurs des revendications 1 à 6, dans lequel l'époxyde polyfonctionnel est choisi parmi le diépoxyde de 1,3-butadiène, le 1,2,7,8-diépoxyoctane, le 1,5-hexadiène d'époxyde, l'éther diglycidylique d'éthylène glycol, l'éther diglycidylique de 1,4-butanediol, l'éther diglycidylique de 1,6-hexanediol, l'éther diglycidylique de polyéthylène glycol, l'éther diglycidylique de polypropylène glycol, l'éther diglycidylique de bisphénol A, de préférence l'éther diglycidylique de 1,4-butanediol, l'éther diglycidylique de polyéthylène glycol et l'éther diglycidylique de polypropylène glycol.

**8.** Procédé selon la revendication 1, dans lequel la réaction est interrompue par l'addition d'un acide concentré jusqu'à neutralité.

**9.** Procédé selon une ou plusieurs des revendications 1 à 8, dans lequel le produit réticulé est purifié par élimination de la phase liquide et lavage jusqu'à disparition des contaminants avec un mélange homogène eau/solvant organique qui n'augmente pas le gonflement de la phase solide.

**10.** Procédé selon la revendication 9, dans lequel le mélange eau/solvant organique est saturé avec du chlorure de sodium dans les premières eaux de lavage.

**11.** Procédé selon la revendication 9 ou 10 dans lequel le solvant organique présent dans le mélange de lavage est l'éthanol ou l'acétone.

**12.** Procédé selon la revendication 9, dans lequel le rapport du solvant organique à l'eau est $\geq 2$.

**13.** Procédé selon une ou plusieurs des revendications 1 à 12, dans lequel le produit réticulé est obtenu par séchage de la matière après le lavage.

**14.** Procédé selon la revendication 13, dans lequel le séchage est obtenu par lavage exhaustif avec un solvant organique anhydre miscible à l'eau et par séchage ultérieur sous vide à une température élevée ou par lyophilisation.

**15.** Matières que l'on obtient via les procédés selon les revendications 1 à 14.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4963666 A
- US 5827937 A
- KR 1020050059521
- US 6921819 B
- US 20050281880 A
- US 20060105022 A
- US 20070026070 A
- US 20070196426 A
- US 6630167 B

**Non-patent literature cited in the description**

- **T. BITTER ; H.M. MUIR.** *Anal. Biochem.,* 1962, 330-334
- **SLATER TF ; SAWYER B ; STRAULI U.** *Biochim Biophys Acta,* 1963, vol. 77, 383-393